(19) Europäisches Patentamt
European Patent Office
Office européen des brevets

(11) Veröffentlichungsnummer: **0 029 940**
**B1**

# (12) EUROPÄISCHE PATENTSCHRIFT

(45) Veröffentlichungstag der Patentschrift: **14.03.84**

(51) Int. Cl.³: **C 07 D 277/82**

(21) Anmeldenummer: **80106961.8**

(22) Anmeldetag: **12.11.80**

## (54) Verfahren zur Herstellung von 2,2'-Imino-bis-benzthiazol-Verbindungen.

(30) Priorität: **24.11.79 DE 2947489**

(43) Veröffentlichungstag der Anmeldung: **10.06.81 Patentblatt 81/23**

(45) Bekanntmachung des Hinweises auf die Patenterteilung: **14.03.84 Patentblatt 84/11**

(84) Benannte Vertragsstaaten: **BE CH DE FR GB IT LI**

(56) Entgegenhaltungen: **US - A - 3 726 889**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr.,** ***Heinrich-Bleicher-Strasse 40, D-6000 Frankfurt am Main 50 (DE)***

**EP 0 029 940 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 2,2'-Imino-bis-benzthiazol-Verbindungen

2,2'-Imino-bis-benzthiazol-Verbindungen sind bekannt. Sie finden in der Technik als Vulkanisierhilfsmittel Verwendung (vgl. US-PS 3 726 889) oder dienen als Ausgangsprodukte für die Herstellung von am Iminostickstoff substituierten 2,2'-Imino-bis-benzthiazolen, wie N,N-Bis-(2-benzthiazolyl)-sulfenamiden, die gleichfalls als Vulkanisierhilfsmittel verwendet werden (vgl. US-PS 3 689 467). — Weiterhin ist aus J. Het. Chem. 12 (1), 37—42 (1975), und Synthesis 1971, 540+541, ein Verfahren zur Herstellung von 2,2'-Imino-bis-benzthiazolen bekannt, bei welchem S,S-Dimethyl-(cyano-imido)-dithiocarbonat mit o-Amino-thiophenolen umgesetzt wird. Diesem Verfahren haften jedoch bei technischer Durchführung verschiedene schwerwiegende Mängel an, so die schwierig zugänglichen Ausgangsprodukte und die Bildung des übelriechenden und giftigen Methylmercaptans.

Mit dieser Erfindung wurde nun ein verbessertes und technisch einfach durchführbares Verfahren zur Herstellung von 2,2'-Imino-bis-benzthiazol-Verbindungen der allgemeinen Formel 1

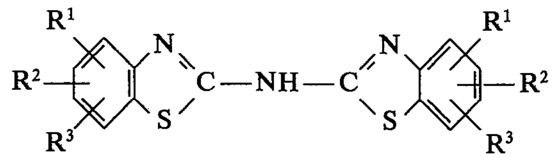

(1)

in welcher $R^1$ ein Wasserstoffatom, einen gegebenenfalls substituierten Alkylrest, einen gegebenenfalls substituierten Alkoxyrest, ein Halogenatom, eine Nitrogruppe oder eine Cyangruppe bedeutet, $R^2$ für ein Wasserstoffatom, einen gegebenenfalls substituierten Alkylrest, einen gegebenenfalls substituierten Alkoxyrest, ein Halogenatom oder eine Cyangruppe steht und $R^3$ ein Wasserstoffatom, einen gegebenenfalls substituierten Alkylrest oder einen gegebenenfalls substituierten Alkoxyrest bedeutet, wobei $R^1$ und $R^2$ und $R^3$ gleich oder verschieden voneinander sein können,

gefunden, das dadurch gekennzeichnet ist, daß man eine 2-Amino-benzthiazol-Verbindung der allgemeinen Formel 2

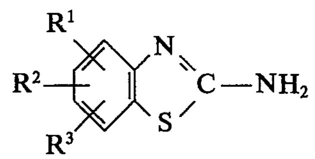

(2)

in welcher $R^1$, $R^2$ und $R^3$ die obengenannten Bedeutungen haben,

entweder bei einer Temperatur von 130°C oder darüber, wie bei 130 bis 210°C, in Gegenwart einer sauren Verbindung, gegebenenfalls in einem Löse- oder Verdünnungsmittel von einem Siedepunkt von oberhalb 125°C, oder in diesem Löse- oder Verdünnungsmittel und ohne eine saure Verbindung bei einer Temperatur oberhalb 200°C erhitzt.

Bei den oben erwähnten Substituenten $R^1$, $R^2$ und $R^3$ sind die Alkylreste vorzugsweise solche von 1 bis 5 C-Atomen, wie insbesondere die Methyl- und Ethyl-Gruppe; substituiert sind sie vorzugsweise durch Alkoxygruppen von 1 bis 4 C-Atomen. Ebenso sind die genannten Alkoxyreste vorzugsweise solche von 1 bis 5 C-Atomen, wie die Methoxy- und Ethoxygruppe; substituiert sind diese ebenso beispielsweise durch eine Alkoxygruppe von 1 bis 4 C-Atomen. Halogenatome sind beispielsweise Fluor und Brom, vorzugsweise Chlor.

Die als Ausgangsverbindung dienende 2-Amino-benzthiazol-Verbindung kann somit gemäß der Erfindung entweder als solche oder in einem über 125°C siedenden Löse- oder Verdünnungsmittel unter der katalytischen Wirkung einer sauren Verbindung miteinander umgesetzt werden. Bei Anwendung der sauren Verbindung liegen die Reaktionstemperaturen vorzugsweise bei 150 bis 185°C. Die saure, insbesondere stark saure, wie mineralsaure, Verbindung kann in katalytischen Mengen oder bis zu stöchiometrischen Mengen im Reaktionsansatz zugegen sein; schwächer saure, insbesondere organische Verbindungen, die als Lösemittel oder Verdünnungsmittel dienen, können auch im Überschuß zugegen sein. Unter katalytischen Mengen werden solche von kleiner als 0,01 Mol, bezogen auf 1 Mol der eingesetzten 2-Aminobenzthiazol-Verbindung, vorzugsweise 0,001 bis 0,01 Mol, bezogen auf 1 Mol dieser Ausgangsverbindung, angesehen.

Die Reaktionszeiten für das erfindungsgemäße Verfahren liegen, abhängig von der eingesetzten Ausgangsverbindung und den gewählten Reaktionsbedingungen, in der Regel zwischen 8 und 20 Stunden.

Saure Verbindungen, die bei der Reaktion eingesetzt werden und diese katalysieren, sind beispielsweise Mineralsäuren, wie Salzsäure, Schwefelsäure, Phosphorsäure und Polyphosphorsäuren, einschließlich der Pyrophosphorsäure, weiterhin auch saure hochsiedende organische Verbindungen, die gleichzeitig als Lösemittel dienen können, wie solche mit phenolischen Gruppen,

wie Phenol, Resorcin, die verschiedenen Kresole, Naphthole, aliphatische und aromatische Carbonsäuren, wie Propionsäure und Benzoesäure.

Eine erfindungsgemäß verwendbare saure Verbindung kann jedoch auch ein saures Salz der als Ausgangsverbindung eingesetzten 2-Aminobenzthiazol-Verbindung sein, das dem Reaktionsansatz der 2-Aminobenzthiazol-Verbindung in katalytischer Menge zugesetzt werden kann. Solche Salze sind die der erwähnten Mineralsäuren, wie das Salz der Salzsäure, Schwefelsäure oder Phosphorsäure. Dieses stark saure Salz einer 2-Aminobenzthiazol-Verbindung kann auch als solches oder in Mischung mit der entsprechenden 2-Aminobenzthiazol-Base zur erfindungsgemäßen Herstellung der 2,2'-Imino-bis-benzthiazol-Verbindung in die Reaktion eingesetzt werden.

Lösemittel oder Verdünnungsmittel, in denen die Reaktion durchgeführt werden kann, sind allgemein bekannte Lösungsmittel mit einem Siedepunkt von oberhalb 125°C, die gegenüber den Reaktionsbedingungen inert sind, wie die Halogenbenzole und Halogentoluole, Halogen-naphthaline, Tetralin, Nitrobenzol und insbesondere aprotische dipolare Lösemittel, wie Dimethylformamid, N-Methyl-pyrrolidon, Dimethylsulfoxid, Formamid, N-alkylierte Acetamide, N-alkylierte Harnstoffe und N-alkylierte Phosphorsäureamide, wie Dimethylacetamid, Tetramethylharnstoff, Tetrabutylharnstoff und Hexamethyl-phosphorsäureamid.

Die erfindungsgemäße Reaktion verläuft unter Umsetzung von 2 Mol der 2-Aminobenzthiazolverbindung unter Abspaltung von Ammoniak, das durch Säure gebunden werden kann. Das Ende der Reaktion kann bei der Verwendung von katalytischen Mengen der sauren Verbindung leicht durch Beendigung der Ammoniakentwicklung, die die Reaktion begleitet, bestimmt werden; setzt man stöchiometrische Mengen der sauren Verbindung ein, so kann das Fortschreiten und das Ende der Reaktion durch eine analytische Überwachung, wie beispielsweise durch die Dünnschichtchromatographie, verfolgt und bestimmt werden. Nach Beendigung der Reaktion läßt man das Reaktionsgemisch abkühlen, verdünnt es gegebenenfalls mit einem hierfür geeigneten Lösemittel, in dem die Imino-bis-benzthiazol-Verbindung wenig löslich ist, oder entfernt das Lösemittel, das im Reaktionsansatz verwendet wurde, beispielsweise mit Wasserdampf, filtriert die hergestellte Imino-bis-benzthiazol-Verbindung ab, wäscht sie gegebenenfalls mit dem entsprechenden Lösemittel nach und trocknet sie. Lösemittel, die zur Ausfällung der erfindungsgemäß hergestellten 2,2'-Imino-bis-benzthiazol-Verbindungen aus der Reaktionsmischung verwendet werden, sind vorzugsweise solche, die in jedem Verhältnis mit den im erfindungsgemäßen Verfahren verwendeten Löse- und Verdünnungsmittel mischbar sind. Gleiches gilt auch für die Lösemittel, mit denen die erfindungsgemäß hergestellten Verbindungen nach der Isolierung nachgewaschen werden. Diese sind beispielsweise niedere aliphatische Alkohole und Ketone.

Die erfindungsgemäß hergestellten 2,2'-Imino-bis-benzthiazol-Verbindungen fallen in reiner Form und hoher Ausbeute an; sie können ohne weitere Reinigung direkt für Folgereaktionen eingesetzt werden.

Die erfindungsgemäße Herstellungsweise, in welcher die Umsetzung in Gegenwart einer sauren Verbindung erfolgt, ist bevorzugt. Zwar erhält man die erfindungsgemäß herstellbaren Verbindungen auch in Abwesenheit der sauren Verbindungen in reiner Form, jedoch müssen wesentlich höhere Temperaturen, nämlich über 200°C, und verlängerte Reaktionszeiten angewendet werden, um gleich gute hohe Ausbeuten zu erhalten.

Insbesondere bevorzugt ist das erfindungsgemäße Verfahren zur Herstellung der Verbindungen der allgemeinen Formel 1 aus den Verbindungen der allgemeinen Formel 2, in welcher $R^1$ für ein Wasserstoffatom, eine Methyl-, Methoxy-, Äthoxy-, $\beta$-Methoxyäthoxy- oder eine Nitrogruppe oder ein Chloratom steht, $R^2$ ein Wasserstoffatom, eine Methyl- oder Methoxy- oder Äthoxygruppe oder ein Chloratom und $R^3$ ein Wasserstoffatom oder eine Methylgruppe bedeutet.

Die nachstehenden Beispiele dienen zur Erläuterung des Erfindungsgegenstandes. Die Teile sind Gewichtsteile, die Prozentangaben beziehen sich auf Gewichtsprozente, sofern nicht anders vermerkt.

## Beispiel 1

Man trägt in 128 Teile geschmolzenes Phenol mit einer Temperatur von 50°C 300 Teile 2-Amino-benzthiazol unter Rühren ein, erwärmt die Reaktionsmischung auf eine Temperatur von 180°C und rührt bei dieser Temperatur 20 Stunden lang. Man läßt abkühlen oder gibt vorsichtig unter Rückflußkühlung 400 Teile Äthanol hinzu, rührt eine Stunde bei etwa 80°C nach, saugt die ausgefallene Verbindung ab, wäscht sie mit Äthanol phenolfrei und trocknet sie.

Man erhält 253 Teile 2,2'-Imino-bis-benzthiazol vom Schmelzpunkt 257 bis 257,5°C entsprechend einer Ausbeute von 89,4% der Theorie. Das Produkt ist analysenrein und kann direkt für Folgereaktionen verwendet werden.

## Beispiel 1a

Man verfährt in der in Beispiel 1 angegebenen Verfahrensweise, verwendet jedoch anstelle von

Phenol eine entsprechende Menge Propionsäure oder o-Kresol oder p-Kresol. Man erhält auch hier das 2,2′-Bis-benzthiazolylamin in gleich guter Ausbeute und Qualität.

### Beispiel 2

Eine Mischung aus 90 Teilen 6-Methoxi-2-aminobenzthiazol, 10,8 Teilen 6-Methoxi-2-aminobenzthiazoliumchlorid und 250 Teilen 1,2,4-Trichlorbenzol wird unter Rühren auf 160 bis 165° C erwärmt; diese Temperatur wird während 18 Stunden unter Rühren gehalten, anschließend das als Lösemittel verwendete Trichlorbenzol mittels Wasserdampfdestillation entfernt. Aus der so erhaltenen, vom Trichlorbenzol befreiten wäßrigen Suspension saugt man das erfindungsgemäß hergestellte 2,2′-Imino-bis-6-methoxibenzthiazol ab und trocknet es. Man erhält es in einer Ausbeute von 90,6% der Theorie mit einem Schmelzpunkt von 271 bis 272° C in praktisch analysenreiner Form.

### Beispiel 2a

Man verfährt in der im Beispiel 2 angegebenen Verfahrensweise, verwendet jedoch anstelle des Trichlorbenzols die entsprechende Menge 1,2-Dichlorbenzol oder Nitrobenzol; man erhält die erfindungsgemäß hergestellte Verbindung in gleich guter Ausbeute und Reinheit.

### Beispiel 3

Man versetzt eine Lösung von 77,6 Teilen 6-Äthoxi-2-aminobenzthiazol in 100 Teilen Dimethylformamid tropfenweise mit 10 Teilen einer 96%igen wäßrigen Schwefelsäure; diese Reaktionsmischung wird während 16 Stunden bei einer Temperatur von 150 bis 160° C gerührt. Anschließend verdünnt man sie mit 500 Teilen von 50%igem wäßrigem Methanol, saugt die ausgefallene Verbindung ab, wäscht sie mit Wasser und trocknet sie. Es werden 71,3 Teile chromatographisch reines 2,2′-Imino-bis-6-äthoxibenzthiazol mit einem Schmelzpunkt von 217,5 bis 218,5° C entsprechend einer Ausbeute von 96,1% der Theorie erhalten.

### Beispiel 3a

Man verfährt, wie im Beispiel 3 beschrieben, verwendet jedoch anstelle des Dimethylformamids als Lösemittel eine gleiche Menge N-Methyl-pyrrolidon oder Tetrabutylharnstoff; man erhält ebenfalls die im Beispiel 3 hergestellte Verbindung in gleich guter Ausbeute und Reinheit.

### Beispiele 4 bis 21

Verfährt man in gleicher Weise zu den in den obigen Ausführungsbeispielen 1, 2 oder 3 beschriebenen Verfahrensvarianten und setzt die in den nachfolgenden Tabellenbeispielen durch die Substituenten gemäß der allgemeinen Formel 2 charakterisierten Ausgangsverbindungen ein, so erhält man die 2,2′-Imino-bis-benzthiazol-Verbindungen entsprechend der allgemeinen Formel 1 mit den in den Tabellenbeispielen angegebenen Substituenten in hoher Reinheit und den sie charakterisierenden Schmelzpunkten und in der angegebenen Ausbeute.

| Bsp. | Analog Beispiel | $R^1$ | $R^2$ | $R^3$ | Ausbeute (% d. Th.) | Smp. (°C) |
|---|---|---|---|---|---|---|
| 4 | 2 | Wasserstoff | Wasserstoff | Wasserstoff | 83,1 | 256–257 |
| 5 | 3 | Wasserstoff | Wasserstoff | Wasserstoff | 91,7 | 257–257,5 |
| 6 | 1 | 4-Methyl | Wasserstoff | Wasserstoff | 93,7 | 213,5–214,5 |
| 7 | 3 | 4-Methyl | Wasserstoff | Wasserstoff | 90,0 | 214–214,5 |
| 8 | 3 | 6-Methyl | Wasserstoff | Wasserstoff | 92,6 | 276–276,5 |
| 9 | 3 | 4-Methoxy | Wasserstoff | Wasserstoff | 75,2 | 181,5–183 |
| 10 | 3 | 6-Methoxy | Wasserstoff | Wasserstoff | 96,0 | 271,5–272,5 |
| 11 | 1 | 6-Äthoxy | Wasserstoff | Wasserstoff | 85,7 | 217–218 |
| 12 | 2 | 6-Äthoxy | Wasserstoff | Wasserstoff | 81,9 | 216,5–218 |
| 13 | 3 | 4-(β-Methoxy)-äthoxy | Wasserstoff | Wasserstoff | 82,0 | 102–105 |
| 14 | 3 | 6-Nitro | Wasserstoff | Wasserstoff | 96,4 | > 300 |
| 15 | 3 | 4-Chlor | Wasserstoff | Wasserstoff | 91,9 | 270–272,5 |
| 16 | 3 | 4-Methyl | 6-Methyl | Wasserstoff | 82,6 | 260–262,5 |
| 17 | 3 | 4-Methyl | 7-Methyl | Wasserstoff | 91,8 | 170–172 |
| 18 | 3 | 4-Methyl | 6-Chlor | Wasserstoff | 80,6 | 273–274,5 |
| 19 | 3 | 4-Methyl | 7-Chlor | Wasserstoff | 92,7 | 252–252,5 |
| 20 | 3 | 4-Methoxy | 6-Chlor | 7-Methyl | 92,0 | 266,5–267,5 |
| 21 | 3 | 4-Methoxy | 7-Methoxy | Wasserstoff | 89,7 | 136–138 |

Beispiel 22

Man erhitzt eine Mischung aus 75 Teilen 2-Amino-benzthiazol und 250 Teilen 1-Chlornaphthalin unter Rühren 40 Stunden lang bei 210°C. Sodann entfernt man das Lösemittel mittels Wasserdampfdestillation und filtriert aus der wäßrigen Sumpfphase das hergestellte 2,2'-Imino-bis-benzthiazol ab. Man wäscht es mit Wasser und trocknet es.

Schmelzpunkt: 256 – 258°C; Ausbeute: 46,6% der Theorie.

**Patentansprüche**

1. Verfahren zur Herstellung von 2,2'-Imino-bis-benzthiazol-Verbindungen der allgemeinen Formel 1

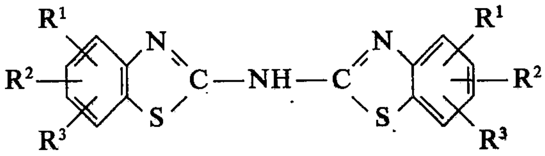

(1)

in welcher $R^1$ ein Wasserstoffatom, einen gegebenenfalls substituierten Alkylrest, einen gegebenenfalls substituierten Alkoxyrest, ein Halogenatom, eine Nitrogruppe oder eine Cyangruppe bedeutet, $R^2$ für ein Wasserstoffatom, einen gegebenenfalls substituierten Alkylrest, einen gegebenenfalls substituierten Alkoxyrest, ein Halogenatom oder eine Cyangruppe steht und $R^3$ ein

Wasserstoffatom, einen gegebenenfalls substituierten Alkylrest oder einen gegebenenfalls substituierten Alkoxyrest bedeutet, wobei $R^1$, $R^2$ und $R^3$ gleich oder verschieden voneinander sein können,
dadurch gekennzeichnet, daß man eine 2-Amino-benzthiazol-Verbindung der allgemeinen Formel 2

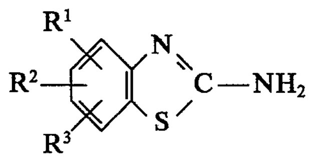

(2)

in welcher $R^1$, $R^2$ und $R^3$ die obengenannten Bedeutungen haben,

a) entweder bei einer Temperatur von 130°C oder darüber in Gegenwart einer sauren Verbindung, gegebenenfalls in einem Löse- oder Verdünnungsmittel von einem Siedepunkt von oberhalb 125°C, oder
b) in diesem Löse- oder Verdünnungsmittel und ohne eine saure Verbindung bei einer Temperatur oberhalb 200°C

erhitzt.

2. Verfahren nach Anspruch 1, dadurch gekennzeichnet, daß das Lösungsmittel ein saures Lösemittel ist.

3. Verfahren nach Anspruch 1, dadurch gekennzeichnet, daß die saure Verbindung in katalytischen Mengen bis zu stöchiometrischen Mengen, bezogen auf die 2-Amino-benzthiazol-Verbindung, im Reaktionsansatz zugegen ist.

4. Verfahren nach Anspruch 1 oder 3, dadurch gekennzeichnet, daß die saure Verbindung Salzsäure, Schwefelsäure, Phosphorsäure, Polyphosphorsäure, Phenol oder ein Kresol ist.

5. Verfahren nach Anspruch 1 oder 3, dadurch gekennzeichnet, daß die saure Verbindung das salzsaure, schwefelsaure oder phosphorsaure Salz der 2-Amino-benzthiazol-Verbindung ist.

6. Verfahren nach Anspruch 2, dadurch gekennzeichnet, daß das Lösemittel Phenol oder ein Kresol ist.

7. Verfahren nach einem der Ansprüche 1 bis 6, dadurch gekennzeichnet, daß bei Variante a) die Reaktionstemperatur 130 bis 210°C ist.

8. Verfahren nach einem der Ansprüche 1 bis 6, dadurch gekennzeichnet, daß bei Variante a) die Reaktionstemperatur 150 bis 185°C ist.

**Claims**

1. Process for the manufacture of 2,2'-imino-bis-benzothiazole compounds of the general formula

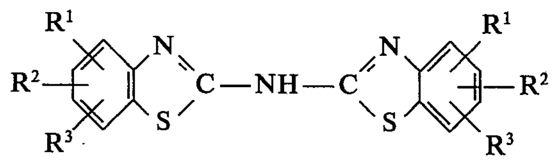

(1)

in which $R^1$ is a hydrogen atom, an optionally substituted alkyl radical, an optionally substituted alkoxy radical, a halogen atom, a nitro group or a cyano group, $R^2$ is a hydrogen atom, an optionally substituted alkyl radical, an optionally substituted alkoxy radical, a halogen atom or a cyano group, and $R^3$ is a hydrogen atom, an optionally substituted alkyl radical or an optionally substituted alkoxy radical, $R^1$, $R^2$ and $R^3$ being identical or different from one another,
characterized by heating a 2-amino-benzothiazole compound of the general formula

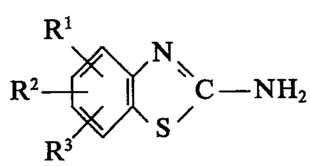

(2)

in which $R^1$, $R^2$ and $R^3$ have the above mentioned meanings,

a) either at a temperature of 130°C or above 130°C in the presence of an acidic compound, optionally in a solvent or diluent having a boiling point of above 125°C, or
b) in such a solvent or diluent and without an acidic compound at a temperature above 200°C.

2. Process according to claim 1, characterized in that the solvent is an acidic solvent.

3. A process according to claim 1, characterized in that the acidic compound is present in the reaction mixture in catalytic amounts up to stoichiometric amounts, relative to the 2-amino-benzothiazole compound.

4. A process according to claim 1 or 3, characterized in that the acidic compound is hydrochloric acid, sulfuric acid, phosphoric acid, polyphosphoric acid, phenol or a cresol.

5. A process according to claim 1 or 3, characterized in that the acidic compound is the hydrochloric, sulfuric or phosphoric salt of the 2-amino-benzothiazole compound.

6. A process according to claim 2, characterized in that the solvent or diluent is phenol or a cresol.

7. A process according to anyone of the claims 1 to 6, characterized in that the reaction temperature in the process variant a) is from 130 to 210° C.

8. A process according to anyone of the claims 1 to 6, characterized in that the reaction temperature in the process variant a) is from 150 to 185° C.

**Revendications**

1. Procédé de préparation d'imino-2,2'-bis-benzothiazoles répondant à la formule générale 1:

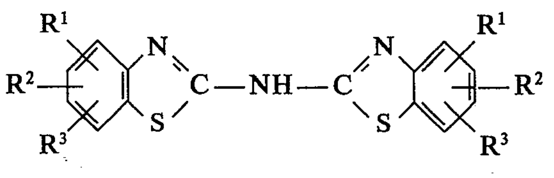

(1)

dans laquelle:

$R^1$ représente un atome d'hydrogène, un alkyle éventuellement substitué, un alcoxy éventuellement substitué, un atome d'halogène, un radical nitro ou un radical cyano,

$R^2$ représente un atome d'hydrogène, un alkyle éventuellement substitué, un alcoxy éventuellement substitué, un atome d'halogène ou un radical cyano, et

$R^3$ représente un atome d'hydrogène, un alkyle éventuellement substitué ou un alcoxy éventuellement substitué,

et dans laquelle $R^1$, $R^2$ et $R^3$ sont identiques ou différents les uns des autres,

procédé caractérisé en ce qu'on chauffe un amino-2 benzothiazole répondant à la formule générale 2:

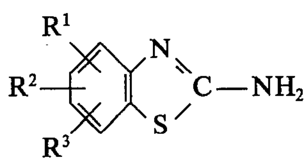

(2)

dans laquelle $R^1$, $R^2$ et $R^3$ ont les significations indiquées ci-dessus, soit:

a) à une température de 130°C ou supérieure, en présence d'un composé acide, éventuellement dans un solvant ou diluant ayant un point d'ébullition supérieur à 125°C, soit

b) dans ce solvant ou diluant et sans composé acide, à une température supérieure à 200°C.

2. Procédé selon la revendication 1 caractérisé en ce que le solvant est un solvant acide.

3. Procédé selon la revendication 1 caractérisé en ce que le composé acide est présent, dans le mélange réactionnel, en une quantité catalytique ou en une quantité plus élevée pouvant aller jusqu'à la quantité stoechiométrique par rapport à l'amino-2-benzothiazole.

4. Procédé selon l'une des revendications 1 et 3, caractérisé en ce que le composé acide est l'acide chlorhydrique, l'acide sulfurique, l'acide phosphorique, un acide polyphosphorique, le phénol ou un crésol.

5. Procédé selon l'une des revendications 1 et 3, caractérisé en ce que le composé acide est le sel formé par l'amino-2 benzothiazole avec l'acide chlorhydrique, l'acide sulfurique ou l'acide phosphorique.

6. Procédé selon la revendication 2 caractérisé en ce que le solvant est le phénol ou un crésol.

7. Procédé selon l'une quelconque des revendications 1 à 6, caractérisé en ce que, dans la variante (a), on effectue la réaction à une température de 130 à 210°C.

8. Procédé selon l'une quelconque des revendications 1 à 6, caractérisé en ce que, dans la variante (a), on effectue la réaction à une température de 150 à 185°C.